# EUROPÄISCHE PATENTANMELDUNG

(11) **EP 1 522 854 A1**
(43) Veröffentlichungstag der Anmeldung: **13.04.2005**
(21) Anmeldenummer: 03022366.3
(22) Anmeldetag: 06.10.2003
(51) Int. Cl.: G01N 33/543, B01L 3/00, G01N 33/552

(54) **Verwendung von eckigen Körpern als Träger von Molekülen oder Substanzen in enzymatischen, immunologischen, molekularbiologischen oder genetischen Analyseverfahren**

(71) Anmelder: DST Diagnostic Science & Technology GmbH, 19061 Schwerin (DE)
(72) Erfinder: Schwertner, Heiko, Dr., 19055 Schwerin (DE)

(57) **Zusammenfassung**

Die Erfindung betrifft den Einsatz von Kantenscheiben als Träger in an sich bekannten enzymatischen, immunologischen, molekularbiologischen oder genetischen Analyseverfahren, insbesondere ELISA Analyseverfahren, um deren bekannte Nachteile zu vermeiden. Kantenscheiben im Sinne der Erfindung sind Körper, die eine durchgängige Ausnehmung besitzen, wie z.B. gerade, runde oder vieleckige Hohlzylinder oder Ringe. Die Kantenscheiben können aus Glas, Kunststoffen, Papier oder Metall bestehen, und können mit allen derzeit verwendeten Reaktionsgefaßen wie Mikrotiterplatten, Mikrotiterplatten-Riegeln oder Einzelkavitäten, Röhrchen usw. eingesetzt werden.

## Beschreibung

### Einleitung

Die Erfindung hat das Ziel, mit einem Träger auf dem Moleküler aller Art gebunden werden können enzymatische, immunologische und molekularbiologische Analyseverfahren durchzuführen. In der Literatur werden solche Träger auch als Festphasen bezeichnet. Diese Träger oder Festphasen werden im folgenden auch Kantenscheibe genannt.

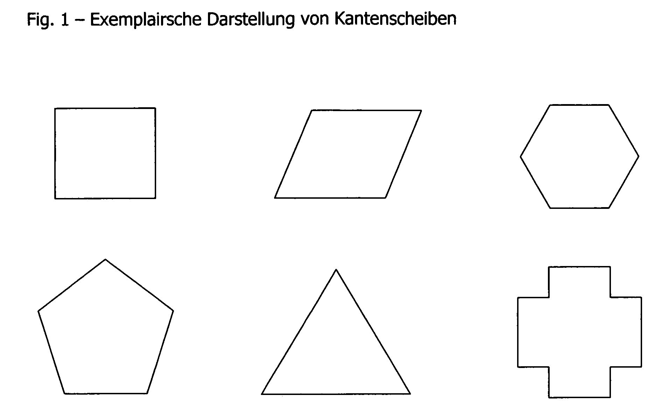

Als Kantenscheibe (KS) werden Körper bezeichnet, deren Form nicht rund ist, sondern Kanten mit Ecken enthält wie Dreiecke, Quadrate, Fünfecke, Secksecke oder Vielecke wie in Fig. 1 Exemplarisch dasgestellt ist, ohne den Patentumfang einzuschränken. Ferner enthält die Kantenscheibe (KS) eine oder mehrere durchgängige Aussparungen.

Die Erfindung betreffenden Kantenscheibe ermöglichen es, alle bekannten Detektionsverfahren und Markierungsverfahren der oben genannten Analyseverfahren wie enzymatische, immunologische, molekularbiologische oder genetische Analyseverfahren zu verwenden.

Enzymatische, immunologische, molekularbiologische Analyseverfahren können zusammenfassend als ELISA (Enzyme Linked Immuno Sorbent Assay) bezeichnet werden. Diese sind eine Standardmethode in der modernen Analytik.

ELISA ist ein weitverbreitetes Labortestverfahren bei dem bioorganische Moleküle nachgewiesen und quantitativ bestimmt werden können. Diese bioorganischen Moleküle können Antigene, Antikörper, Hormone, Hormonrezeptoren, biochemische Botenstoffe, Medikamente, Drogen und andere Analyten in den Körperflüssigkeiten von Mensch und Tier sein. Die ELISA Verfahren haben sich auch als nützlich für den Nachweis und die Bestimmung von verschiedenen unverträglichen Umweltsubstanzen wie Pestizide, Insektizide im Wasser, Boden und in Lebensmitteln bewährt. Gerade in der Lebensmittelüberwachung werden ELISA Techniken eingesetzt um verbotene Additive wie Masthormone, organische Giftstoffe, Mykotoxine nachzuweisen und quantitativ zu bestimmen.

Der Begriff Träger bezieht sich auf eine stabile, während der Reaktion innerte Substanz, wie zum Beispiel Kunststoffe (Polysterole, Nylon, Polyacryl, Polystyrole etc.), Papier, Glas oder Keramik. Diese Träger muß zusätzlich chemisch oder physikalisch aktivierbar sein.

Es sind eine Vielzahl von Verfahren bekannt, mit denen eine Aktivierung oder chemische Modifizierung dieser Trägern möglich ist, für die Auswahl des verfahrens ist das Material aus dem der Träger besteht von Bedeutung. Diese Aktivierungen oder chemische bzw. physikalische Modifizierungen haben das Ziel, daß Substanzen an diesen Oberflächen gebunden (meist kovalent) werden können. Nach der Aktivierung trägt die aktivierte Träger trägt auf ihrer Oberfläche Bindungsstellen die fähig sind, bioorganische Moleküle wie Antigenmoleküle (AG), meist kovalent, zu binden. Ebenso können Antikörper, Hormone, Hormonrezeptoren, biochemische Botenstoffe, Medikamente, Drogen etc. können ebenfalls gebunden werden.

Durch die Bindung wird eine räumliche Fixierung erreicht, mit der man in der Lage ist verschiedenen Schritte wie Waschungen, Inkubationen, Messungen, etc. durchzuführen, ohne das es zu einer Vermengung der gebundenen Substanz (Bioorganische Moleküle wie Antigene) mit der Reaktionslösung kommt. Somit ist man in der Lage die an dem Träger gebundene Substanz mit Flüssigkeitsproben wie Serum; Urin, oder anderen Probenmaterialien zu inkubieren. Dort findet eine meist bekannte und gewünschte Reaktion zwischen der oder den gebundenen und den sich in Lösung befindlichen Substanzen statt. Auf diese Weise können z.B. im Serum vorhandene spezifische primäre Antikörper aus einem Gemisch gebunden werden.

Nach dieser ersten Inkubation findet mindestens eine zweite Inkubation statt. Diese zweite Inkubation wird in der Regel mit einer Detektor haltigen Lösung durchgeführt. Nach jeder Inkubation wird ein Waschvorgang unternommen, um überflüssige Moleküle zu beseitigen. Als Detektor können z. B. markierte sekundäre Antikörper dienen. Diese können unter anderem mit Enzymen (E) oder einem Farbstoff (F) gekoppelt sein. Die Enzyme erzeugen ein meßbares Signal. Allgemein dienen die Detektormoleküle dazu ein meßbares Signal zu erzeugen. Dabei ist die Menge der erzeugten Signale proportional der Konzentration der pAB. Auf diese Weise läßt sich die Konzentration der pAB bestimmen. Die erzeugten Signale müssen auf den verwendeten Detektor abgestimmt sein, so können Farbmoleküle oder Strahlungen wie UV, IR, radioaktive-, fluorescence-, oder chemolumenescence Strahlungen verwendet werden.

### Stand der Technik

Nach dem heutigen Stand der Technik werden analytische Techniken wie ELISA oder andere analytische Methoden wie enzymatische, immunologische, molekularbiologische oder genetische Analyseverfahren, auf verschiedenen Trägern durchgeführt. Die Form der Trägern variiert stark. Als Trägernformen gibt es eine Vielzahl von Ausführungen, so werden z.B. Röhrchen (verschiedener Größen), Mircotiterplatten (MTP), Kugeln oder Scheiben verwendet. In der Praxis haben sich insbesondere die Mircotiterplatten (MTP) durchgesetzt, für die es eine große Anzahl von Geräten aller Art gibt. In praktisch jedem Labor ist eine vollständige Ausrüstung zur Durchführung von Tests in MTP vorhanden. Diese Trägern werden dazu genutzt Analyten oder Nachweissubstanzen zu binden. Die Art der Bindung ist je nach Einsatzgebiet verschieden, so werden z.B. kovalenten Bindungen oder elektrostatische Bindungen ausgenutzt.

In der Praxis haben sich neben Röhrchen, insbesondere die Mircotiterplatten (MTP) durchgesetzt, für die es eine große Anzahl von Geräten aller Art gibt. Mircotiterplatten bestehen aus kleine miteinander verbundenen Bechern auch Kavitäten (Englisch auch Well) genannt. Ein Standardmaß einer MTP sind 8 mal 12 Kavitäten, mit einem Gesamtvolumen von 300 pl pro Kavität. Die Fläche aus 8 mal 12 Kavitäten wird MTP genannt. Die Kavitäten werden sowohl als reines Reaktionsgefäß, als auch als Träger (innere Oberfläche) für ELISA Techniken verwendet. Weitere Größen und Forem befinden sich im Handel.

Die geometrische Form der MTP Kavitäten hat sich als sehr nützlich für die Durchführung von ELISA's erwiesen. Der Hauptvorteil der Brunnen liegt darin, daß ein Teil der inneren Oberfläche auch als Träger genutzt werden kann und der gesamte Brunnen als Reaktionsgefäß. Es sind eine Vielzahl von Geräten entwickelt worden, um speziell mit MTP zu arbeiten. Für MTP gibt es Waschgeräte, Schüttelgeräte, Meßgeräte mit diversen Detektoren oder Inkubatoren.

Die Möglichkeit der Nutzung der inneren Oberfläche der Kavitäten einer MTP als Träger ist jedoch gleichzeitig ein wesentlicher Nachteil der MTP, wie auch bei den Röhrchen. Es ist notwendig jeden einzelnen Becher der MTP oder jedes Röhrchen zu beschichten. Es ist extrem aufwendig eine identische Belegung mit Molekülen zu erreichen, was in der Praxis sogar fast unmöglich ist. Diese indentische Belegung ist aber notwendig, um die Varianzen innerhalb der Tests so gering wie möglich zu halten. Schon ein Unterschied von 1 % in der Belegungsdichte oder Oberfläche wirkt sich signifikant in der Varianz bei den Testergebnissen aus. Dies ist für einen qualitativ hochwertigen Test nicht akzeptabel, zumal in der Gesamtdurchführung eines Test sich weitere Varianzen addieren (Pipettierfehler, Konzentrationsfehler, etc.). Zur identischen Behandlung der Kavitäten wurde eine Reihe sehr aufwendiger und teurer Geräte entwickelt. Ferner wird die Oberfläche der innen Seite der Kavität (oder des Röhrchens) komplett belegt, was die spätere Messung behindert, da durch diese individuelle Schicht hindurch gemessen wird. Inbesondere bei Test in der Allergiediagnostik ist dies Problematisch, da nicht einheitliche Belegungslösungen verwendet werden können.

Aus diesem Grund wurden Träger beschrieben, die in die MTP oder Röhrchen hineingegeben werden, wie Träger in Form von Kugeln, Partikel oder Scheiben, so wurden auch Scheiben in Form von Sechsecken aus Papier verwendet. Diese lassen sich mit einfachen Methoden nahezu identisch mit Molekülen aller Art belegen, indem die gesamten Körper (mehrere tausend sind möglich) in die Beschichtungslösung geben werden können. Ein Nachteil dieser Körper ist es, daß nach dem transferieren des Körpers in die MTP-Kavitäten ein Detektionsstrahl (IR, UV, etc.) immer durch das Material des Trägernkörpers beeinflußt wird, so daß eine Messung mit dem Körper in der Lösung in der MTP nicht möglich ist. Deshalb müssen diese Arten von Trägern aus den Kavitäten entfernt werden, bevor die eigentliche Messung stattfinden kann.

Es ein Verfahren bekannt, das diesen Nachteil umgeht. Bei diesem Verfahren haben die Träger die Form einer Scheibe (aus dem Material Papier). Diese Scheiben sind mittig mit einem Loch versehen, im nachfolgenden Lochscheiben genannt (Patentnummer P 41 23 324.7). Laut der Patentschrift soll es durch die mittige Öffnung ermöglicht werden, durch die Scheibe hindurch, Licht mit nur geringen Verlusten zu senden. Bei diesen Lochscheiben muß Verfahrens bedingt, ein Kompromiß zwischen maximaler Oberfläche, maximaler Öffnungsweite und noch vertretbarer Stabilität des Trägermaterials eingegangen werden. Dies führt automatisch dazu, daß die Öffnungsweite geringer eingestellt werden muß, als es wünschenswert ist.

Die Nachteile der beschriebenen Verfahren liegen auf der Hand. Bei Trägern ohne Lochung, ob als Fläche oder Körper, ist man nur in der Lage Licht oder Strahlung ohne Verlust an Energie an den Körpern vorbei zu leiten. Wird die Strahlung oder das Licht dennoch durch die Träger geleitet, hat dies immer Veränderungen der Eigenschaften der Strahlung oder des Lichtes zur Folge.

Werden flache Träger wie Scheiben mit Lochung verwendet, so kann Licht mit nur geringen Verlusten durch die Lochung gesandt werden. Nachteil dieser Methode ist es das die Lochung nur einen beschränkten Teil der Fläche einnehmen darf. Der andere Teil der Fläche wird dazu benötigt, den jeweiligen gewünschten Stoff zu binden. Deshalb wird trotz der Lochung ein großer Teil der Strahlung absorbiert oder was schwieriger zu Handhaben ist verändert z.B. durch Streuung, Änderung der Wellenlänge oder der Polarisation etc. Ferner ist nicht gewährleistet, daß die gelochte Scheibe, gleich welchen Materials, sich mittig im Strahlengang befindet, um ein Maximum an Strahlungsenergie unverändert hindurch zulassen. Dies führt dazu, daß ein gewisses Maß an Meßwertschwankungen in Kauf genommen wird. Die Addieren sich mit den weiteren Meßwertschwankungen eines Analyseverfahrens.

Bei beiden Varianten der externen Träger (mit oder ohne Ausnehmung) sind die notwendigen Waschungen zur Durchführung von analytischen Tests nach Aufgabe des Proben materials, nur schwierig zu lösen , da Unter- und Oberseite gewaschen werden müssen. Dies führt in der Praxis dazu, dass häufige und sehr intensive Waschprozeduren angewendet werden. Dies führt zu einer Zeitverlängerung, erheblichen Verbrauch an Waschmittel oder Reagenzlösungen und an Verlust von Testgenauigkeit/-Qualität.

Die beschriebenen Nachteile haben dazu geführt, daß die externen Träger inkl. Lochscheiben sich in der Praxis nicht haben durchsetzen können. Alle die zuvor beschriebenen Trägernkörper können nicht mit vertretbaren Aufwand zur Verwendung in vollautomatisierbaren Verfahren eingesetzt werden. Zwar gibt es entsprechende Automaten (Pipettierautomaten), doch sind diese teuer und haben erhebliche Nachteile, da sie Fehler behaftet sind.

Nicht bekannt sind eckige Körper mit einer oder mehreren rundern oder eckigeren Ausnehmungen, die als Träger für Substanzen oder Moleküle in analytischen Verfahren eingesetzt werden und sind deshalb auch nicht Stand der Technik.

### Vorteile der Erfindung

Der erfindungsgemäße Einsatz von Kantenscheiben (KS) mit durchgängiger Aussparung bietet mehrere Vorteile. Eine solche Kantenscheibe ermöglicht es, daß Strahlung oder Licht verschiedener Wellenlänge oder Polarisation durch den Kantenscheibe geleitet werden kann, ohne daß das Randmaterial die Eigenschaften der Strahlung beeinflußt. Die Kantenscheibe (KS) besitzt eine große Öffungsweite und muß somit nicht so exakt mittig in den Strahlungsgang gebracht werden.

Erfindungsgemäße Kantenscheibe gleich welcher Form (vergl. Fig 1) (dreieckig oder vieleckig) und Querschnittsweite, benötigen keine besonderen und aufwendigen Herstellungsverfahren. Solche KS können mit einfachen Mitteln des Stands der Technik aus verschiedenen Materialien gefertigt werden. Es können ferner alle heute bekannten und auch zukünftige Trägermaterialien zum Aufbau solcher Kantenscheiben herangezogen werden.

Kantenscheiben können auch als Hohlzylinder ausgeführt werden, wobei dann die Kantenhöhe nicht mehr vernachlässigt werden kann und können dadurch aufgrund ihrer einfachen Bauweise in vielen bestehenden Reaktionsgefäßen insbesondere MTP Kavitäten verwendet werden. Somit können alle vorhandenen Analysegeräte und

Verfahren eingesetzt werden, ohne diese speziell anpassen zu müssen, was bei dem derzeitigen Trägern der Fall ist. Dafür sind keine Änderungen der Analysemethode oder der Analysegerätschaft notwendig.

Die erfindungsgemäße Kantenscheibe kann unter anderem aus magnetischem Material, wie Metall Kantenscheibe, Kunststoffe (z.B. Polyacrylamid, Nylon, Polyacryl etc.) mit eingebetteten Eisenpartikeln oder induktiven Material wie elektrisch leitender Kunststoffen oder Aluminium gefertigt werden, so dass einfache und weitläufig bekannte elektromagnetische Transportverfahren Verwendung finden können. Diese Eigenschaft ermöglicht eine vollständige Automation von Analysemethoden, was mit den derzeit bestehenden Verfahren nicht möglich ist. In der allgemein zugänglichen Literatur sind keine aus derartigen Materialien gefertigten Kantenscheibe beschrieben, die bei enzymatische, immunologische und molekularbiologische Analyseverfahren Verwendung finden und zur Automation eingesetzt werden.

Die Oberflächen von modifizierten Metallkantenscheiben besitzen durch Ihre Dichte zwei weitere Vorteile. Zum einen bedingt durch ihre große spezifische Dichte sinken diese in wäßrigen Medien schnell auf den Boden des Analysegefäßes und können somit ausreichend benetzt werden. Zum zweiten lassen sich metallische Kantenscheibe durch elektromagnetische Kräfte bewegen, z.B. in Drehung versetzen. Durch Drehung der Kantenscheibe um die eigene Achse ist man in der Lage eine vollständige Durchmischung berührungsfrei zu erzielen. Das hat zur Folge, daß unerwünschte Kontaminationen während des Analysevorganges aus geschlossen werden können. Solche Metallkantenscheiben lassen sich über deren induktive Eigenschaften erwärmen. Diese Eigenschaft kann bei Analyseverfahren dazu ausgenutzt werden konstante Temperaturen zu erzeugen oder Temperatursprünge erzeugen, wie bei sogenannten PCR-Verfahren Anwendung finden.

Erfindungsgemäße Kantenscheiben können während eines Assays mit einfachen Mitteln gespült werden oder es können auch andere Operationen durchgeführt werden. Damit ist man in der Lage einen Assay auf Basis von Kantenscheiben schneller und kostengünstiger durch zuführen. Ferner wird durch die erfindungsgemäßen Träger sowohl Zeit, als auch Reagenzien eingespart und den Ausschluss und die verringerung von Fehlerquellen die Gesamtqualität der Testverfahren erhöht.

### Ziel der Erfindung

Die der Erfindung zugrunde liegende Aufgabe besteht darin in einem Träger, die Nachteile der bestehenden Trägernformen auszuschließen oder zu minimieren und deren Vorteile beizubehalten. Als Vorteile bestehender Träger können folgende Eigenschaften betrachtet werden:
- Universelle Einsetzbarkeit in alle Arten von Gefäßen (MTP, Röhrchen etc.)
- Massenproduktion mit entsprechender Einheitlichkeit ist möglich
- Verwendbar für viele Kopplungsverfahren

Als Nachteile bestehender Träger müssen folgende Eigenschaften betrachtet werden:
- Höherer Zeitbedarf
- Großer Verbrauch an Wasch-, Lösungsmitteln und Reagienzien
- Reduktion von Testqualität
- Beeinflussung von Messungen
- Transferrierung zur Messung ist notwendig
- Automatisierung ist nut mit erhöhtem Aufwand möglich
- Nicht universell einsetzbar

Die bei der Erfindung verwendeten Kantenscheibe, schließen die Nachteile der beschriebenen Verfahren aus oder verringern sie nachhaltig, wobei die Vorteile von Einzelträgern erhalten bleibt. Ferner sollten die Ziele mit einfachen, extrem kostengünstigen Mitteln erreicht werden. Was durch die erfindungsgemäße Kantenscheibe oder Kantenzylinder gewährleistet wird.

### Ausführung und Beschreibung der Erfindung

Die Aufgabe wird erfindungsgemäß durch die Verwendung von Kantenscheiben gelöst, im Nachfolgenden auch Kantenscheibe (KS) genannt. Als Kantenscheibe (KS) werden Körper bezeichnet, deren Form nicht rund ist, sondern Kanten mit Ecken enthält wie Dreiecke, Quadrate, Fünfecke, Secksecke oder Vielecke wie in Fig. 1 Exemplarisch dasgestellt ist, ohne den Patentumfang einzuschränken. Ferner enthält die Kantenscheibe (KS) eine oder mehrere durchgängige Aussparungen. Ist die Kantenhöhe, gegenüber dem Durchmesser des Trägers, nicht mehr zu vernachlässigen, so werden diese Träger auch als Kantenzylinder bezeichnet. Diese Träger können in allen bekannten Materialien oder Materialmischungen ausgeführt werden, ferner können magnetisierbare oder magnetische Beimengungen in den Materialien oder Materialmischungen beigefügt werden.

Die Höhe oder die Querschnittsweite der Kantenscheiben oder-zylinder können variabel dabei sein. Ein Ring ist dem Kantenscheibe nicht gleich zusetzten, da der Ring einen runden Querschnitt besitzt.

Die erfindungsgemäßen Kantenscheibe oder -zylinder können aus Kunststoffen (z.B. Polyacrylamid, Nylon, Polyacryl, Polystyrol etc.), Glas, Keramik, Papier oder Metall oder anderen bekannten Materialien gefertigt werden. Ferner können die Kantenscheibe aus Kunststoffen (z.B. Polyacrylamid, Nylon, Polyacryl, Polystyrol etc.), Glas oder Papier gefertigt werden in denen magnetisierbare Metallteilchen fest eingelagert sind oder die Oberfläche kann mit solchen Teilchen beschichtet worden sind.

In Experimenten wurde erkannt, dass Kantenscheiben die Ziele der Erfindung lösen, was technisch nicht naheliegend ist, denn in der Praxis werden Träger für analytische Verfahren intensiven Waschprozeduren unterworfen, müssen transferiert werden oder sind mechanisch nicht stabil, was eine Reihe von Nachteilen, welche zuvor beschrieben worden sind, hat.

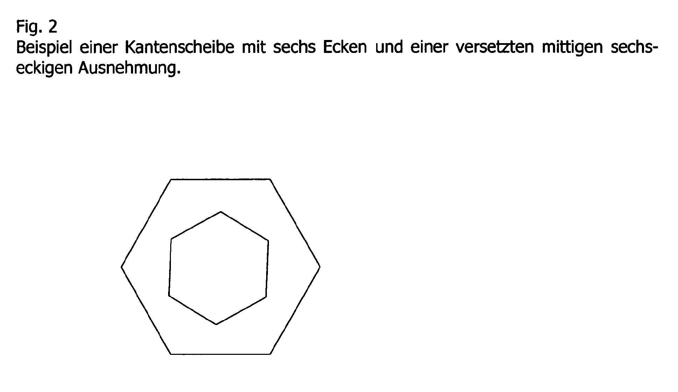

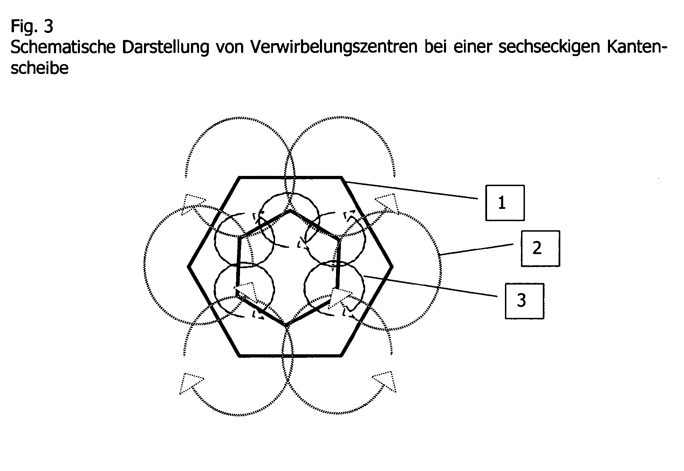

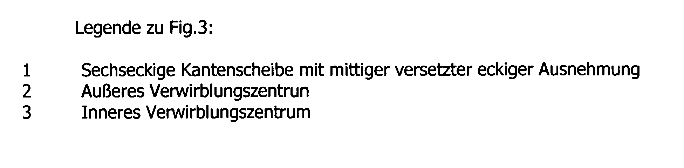

Kantenscheiben sind in der Aufsicht drei oder Mehreckig, sie besitzen eine oder mehrere durchgängige Ausnehmungen (Lochung(en)). Als besonders Effektiv hat sich eine eine drei oder mehreckige Ausnehmung erwiesen. Durch die Ecken werden bei Befüllvorgängen oder Waschvorgängen nahe bei der Trägeroberfläche Verwirbelungen erzeugt siehe Fig.2. Durch die durchgehende Aussparung werden im inneren teil der Fläche der Kantenscheiben oder -zylinder ebenfalls Wirbel erzeugt (Fig.2). In der Summe ergibt sich eine flächendeckende Verwirbelung. Intensität und Effektivität kann über die Gestaltung der Kanten und der durchgehenden Aussparung stark beeinflußt werden.

Diese Wirbelentstehung sorgt für eine schnellere, intensivere und effektivere Austausch von Flüssigkeiten, sowie verbessert die Durchmischungsmöglichkeit. Ferner sorgen die Wirbel für eine Drehung der Träger um die vertikale und horizontale Mittelachse des Trägers, was zusätzlich für einen Austausch von Flüssigkeiten bei der "verdeckten" Oberfläche (Oberfläche die dem Boden des Gefäßes zugewandt ist) sorgt. Deshalb kann auf intensive Wasch-, Reaktions- und Inkubationsprozeduren verzichtet werden. Experimentell konnten solche Zeiten auf ein Minium von weniger als 10 Sekunden reduziert werden, im Gegensatz zu derzeit meist mehrere Minuten andauernden Prozeduren. Dies beschleunigt den gesamt Testablauf, erhöht die Testqualität (durch ausschluss von Fehlerquellen) und spart Wasch-, Reagenz-, und

Lösungsmittel. In der Summe werden dadurch nicht nur die Qualtität eines Tests erhöht, sondern auch die Wirtschaftlichkeit sowie indirekt auch die Umwelt geschont.

Die Vorteile der alten Träger, wie sie zuvor beschrieben worden sind, bleiben erhalten.

Dies ist bei runden oder kugelförmigen Trägern nicht der Fall, auch bei den sechseckigen Papierträgern der Industrie, findet sich ein derartiger Effekt sich intern verstärkenden Wirbel nicht. In der Praxis "kleben" die sechseckigen Papierträger an Boden eines Gefäßes fest. Was durch nachteilige weil intensive Wasch- und Inkubationsprozeduren kompensiert werden muss, wie oben beschrieben worden ist, dies ist die nahe liegende technische Lösung, welche auch in der Praxis verwendung findet. Auch runde Scheiben mit zentraler Ausnehmung haben keine derartigen Effekt, da durch die Rundung bedingt, keine Verwirbelung erzeugt wird, was die oben bei den erfindungsgemäßen Trägern beschriebenen positiven Effekte erzeugt. Deshalb haben runde Scheiben mit Ausnehmung nur den Vorteil, das diese nicht mehr aus dem Reaktionsgefäß entnommen werden müssen. Nachteilige Randeffekt für durchgeleitetes Licht oder Strahlung bleiben. Runde Träger sind u.a. in den Patentschriften DE 42 08 156 A1, DE 4123324.7 DE 19506802 A1 bzw. DE 2801026 C2 beschrieben.

Strahlung bzw. Licht kann durch den Kantenscheibe hingegen ohne Verlust oder anderer nachteiliger Effekte geführt werden. Dadurch kann ein Kantenscheibe aus farbigen oder Licht undurchlässigem Material gefertigt werden. Hiermit werden nachteilige Effekte wie inneres Streulicht oder äußeres Licht stark vermindert oder verhindert. Wird andererseits für den Kantenscheibe klares Material verwendet und die Einstrahlung des Meßlichts/strahlung seitlich vorgenommen, so wird eine definierte Schichtdicke erhalten. Auch dann wenn, sich Flüssigkeiten oder Gase in dem Gefäß befinden.

Weiterhin haben Trägern in Form von Kantenscheiben gegenüber MTP Kavitäten oder anderen Gefäßen, die nur innen beschichtet werden, eine wesentlich vergrößerte Oberfläche. Mathematisch muß die Kantenscheibe als Zylinder betrachtet werden. Physikalisch betrachtet kann die Höhe jedoch vernachlässigt werden, da sie nur einen sehr kleinen Teil zu Gesamtoberfläche beiträgt. Dieser Zylinder kann auf der gesamten Oberfläche beschichtet werden. Bei einer MTP oder anderen Gefäßen kann jedoch zur Beschichtung nur die Bodenfläche und ein Teil der Seitenfläche herangezogen werden. Anderenfalls ergeben sich große Schwankungen in den Testergebnissen, da sonst identische Oberflächenbeschichtungen nicht zu erreichen sind. Wird die zur Beschichtung nutzbare Oberfläche von MTP mit der Oberfläche von Kantenscheiben verglichen so ergibt sich ein Verhältnis von ca. der 3 fachen Oberfläche.

Erfindungsgemäße Kantenscheibe lassen einen kontinuierlichen Strahlengang zu, damit sind kinetische Messungen, einer auf der Oberfläche stattfindenden Reaktion möglich und einfach durchzuführen.

Eine erfindungsgemäße Kantenscheibe ist universell einsetzbar und deshalb kann in den derzeit verwendeten Reaktionsgefäßen wie Microtiterplatten (MTP), Probenröhrchen oder ähnliches eingesetzt werden, ohne daß Änderungen des apparativen Analyseaufbaus notwendig sind. Gerade für den Einsatz in MTP sind zylindrische Kantenscheibe geeignet, da sie in ihrer Bemaßung so angepaßt werden können, daß sie genau in eine Vertiefung (Kavitäten) passen. Kantenscheiben mit einem Durchmesser von 5 mm sind in alle derzeitigen standardmäßig verwendeten Gefäßtypen einzusetzen.

Durch eine nachträgliche Einführung eines Bodens und oder eines Deckels an die erfindungsgemäßen Kantenscheiben, ist man in der Lage aus einen Kantenscheibe ein Gefäß zu gestalten. Was eine Erweiterung der sowieso schon vielseitigen Verwendung ermöglicht, z.B. durch Einführung einer Membran, so daß der so modifizierte Kantenscheibe z.B. zur Filtration eingesetzt werden kann. Kantenscheibe können während eines Assays mit einfachen Mitteln mehrfach gespült bzw. gewaschen oder es können weitere mechanische Operationen durchgeführt werden. Damit ist man in der Lage einen Assay auf Basis von Kantenscheiben schneller und kostengünstiger durchzuführen.

Ein Kantenscheibe kann, nach der Zugabe einer zu analysierenden Reaktionslösung, in der Reaktionslösung verbleiben und muß nicht aus der Lösung transferiert werden, was die Durchführung eines Assays wesentlich beschleunigt und eine Automatisierung ermöglicht. Es ergibt sich also eine zweifache Beschleunigung, einmal dadurch das die Kantenscheibe nicht transferiert werden muss und zu anderen durch den selbstreinigungs Effekt während Wasch- und Reaktionsprozeduren.

Wird die Kantenscheibe aus magnetisierbarem Metall oder Kunststoff mit eingebetteten Metallen gefertigt, so ergeben sich noch weitere Vorteile. Metalle besitzen eine hohe Dichte was dazu führt, daß die Kantenscheibe in den Reaktionslösungen schnell zu Boden sinken. Dies sorgt dafür, daß die Oberfläche der Körper immer benetzt ist. Da Metalle nicht lichtdurchlässig sind, wird für eine wirkungsvolle Abschirmung von Fremdlichtquellen gesorgt, die negativen Einfluß auf eine Messung haben können.

Kantenscheibe aus magnetisierbarem Metall können direkt zum Transport innerhalb eines Analyseautomaten herangezogen werden, in dem mit Elektromagneten ein Greifer aufgebaut wird. Damit ist eine einfache Vollautomatisierung eines Assays realisierbar, was mit den bisherigen Trägern nicht der Fall war.

Rein metallische oder Kantenscheibe oder -zylinder aus Kunststoff mit Eisenpartikeln, lassen sich durch elektromagnetische Kräfte bewegen, z.B. in Drehung versetzen. Durch Drehung der Kantenscheibe um die eigene Achse ist man in der Lage eine vollständige Durchmischung der Reaktionslösung, berührungsfrei, zu erzielen und die Wascheffekte durch die Ecken der Scheibe verstärken. Das hat zur Folge, daß unerwünschte Kontaminationen während des Analysevorganges aus geschlossen werden können und unerwünschtes Konzentrationsgefälle innerhalb der Reaktionslösungen zusätzlich vermieden werden können. Ferner läßt sich Metall über eine elektromagnetische Induktion erwärmen. Diese Eigenschaft kann bei Analyseverfahren dazu ausgenutzt werden konstante Temperaturen oder Temperatursprünge zu erzeugen, so dass Inkubationsschritte eingespart werden können. Dies ergibt neben den zwei vorgenannten einen dritten Vorteil der erfindungsgemäßen Kantenscheiben.

Werden in erfindungsgemäße Träger kunststoffe mit eingelagerten metallpartikeln verwendet, so reicht eine Konzentration von 10 % Metallanteil aus, um die zuvor beschrieben Effekt oder Möglichkeiten zu erzielen.

Eine Kantenscheibe läßt sich in Apparaturen derart einspannten, daß aus ihm direkt eine Meßzelle wird. Diese Apparaturen können sehr einfach auf gebaut werden.

Zusammenfassend ergeben folgende wesentliche Vorteile bei der erfindungsgemäβen Verwendung von kantigen Hohlkörpern in Form von Kantenscheiben oder Kantenzylindern verschiedener Querschnittsformen oder Querschnittsweiten:
1. KS haben eine große Oberfläche
2. Zeitersparnis
3. Materialersparnis an Wasch-, Reaktions- und Lösungsmitteln
4. Eine freier Strahlengang ist möglich
5. Tausende KS können mit einer identischen Beschichtung versehen werden.
6. MTP werden nur als Reaktionsgefäße verwendet, deren angepaßten Analysegeräte können ohne technische Änderung übernommen werden.
7. KS können in allen Reaktionsgefäßen wie Röhrchen etc. eingesetzt werden.
8. Einfache Herstellungsmöglichkeiten von KS
9. Die Verwendung von Metallkantenscheiben (mKS) ermöglicht eine Vollautomation.
10. mKS ermöglichen eine berührungsfreie Bewegung oder Erwärmung über Elektromagnetismus.
11. KS lassen sich als Reaktionskammern verwenden.
12. Kosteneinsparung für den Anwender.
13. Umweltschonend
14. Universell einsetzbar
15. Standardgeräte können verwendet werden

Ein Weg zur Herstellung der erfindungsgemäßen Kantenscheibe z.B. von geraden runden Hohlzylindern oder Ringen besteht darin, daß übliche käufliche eckige Röhren die aus verschiedenen Materialien gefertigt sein können, senkrecht zur Längsrichtung und entsprechend der gewünschten Höhe des Kantenscheibes durchtrennt werden. Durch dieses Verfahren ist eine Massenproduktion solcher Kantenscheibe möglich. Ferner ist es möglich aus Blechen, Folien etc. die KS herauszustanzen. Ein weiteres Verfahren ist der Formenguß eines KS.

Anschließend können die Kantenscheibe auf übliche Weise chemisch aktiviert werden und die in innerten Puffern gelösten Substanzen oder Komponenten an diese gebunden werden. Nach einem Waschvorgang werden verbliebene aktive Restgruppen zumeist mit Ethanolamin deaktiviert bzw. neutralisiert, wonach die erfindungsgemäßen Kantenscheibe gebrauchsfertig sind. Sie können nun in enzymatischen, immunologischen und molekularbiologischen Analyseverfahren eingesetzt werden.

Die erfindungsgemäßen Kantenscheibe aus Metall oder anderen nicht direkt aktivierbaren Materialien wie Glas oder Keramik, welches nicht direkt zur Bindung von gelösten Substanzen oder Komponenten chemisch oder physikalisch aktivierbar ist, wird zuvor mit einer chemisch oder physikalisch aktivierbaren Schicht beschichtet. Diese Schicht wird an in der oben beschriebenen Weise chemisch oder physikalisch aktiviert.

Ein Weg zur Herstellung dieser erfindungsgemäßen Metallkantenscheibe ist das ein Kunststoff (z.B. Polyacrylamid, Nylon, Polyacryl oder andere) oder Materialien in Lösung gebracht werden. Die MetallKantenscheibe werden in diese Beschichtungslösung eingetaucht. Nach einer Reaktionszeit x werden die Körper aus der Lösung entfernt und das Lösungsmittel abgedampft. Die auf der Oberfläche verbliebene Schicht des gelösten Kunststoffes kann jetzt chemisch oder physikalisch modifiziert werden. Die Anlagerung von gelösten Kunststoffen wird durch eine angeraute Oberfläche erleichtert. (Hinweis: Fertige Kunststofflösungen sind käuflich zu erwerben).

Alle Formen der Kantenscheibe können durch eine Mechanik transportiert werden. Dabei können sowohl Saug-, als auch Greifermechanismen verwendet werden. Die Verwendung von Kantenscheiben aus Metall bzw. aus Kunststoffen (z.B. Polyacrylamid, Nylon, Polyacryl etc.), Glas oder Papier in denen magnetisierbare Metallteilchen eingelagert sind, ermöglicht den einfachen Einsatz von elektromagnetischen Transportverfahren. Indem einfache Transportarme mit Elektromagneten für den Transport eingesetzt werden.

Eine Kantenscheibe läßt sich in Apparaturen derart einspannten, daß aus ihm direkt eine Meßzelle wird. Diese Apparaturen können sehr einfach auf gebaut werden wie es in der die Zeichnung ausgeführt ist.

## Patentansprüche

1. Mehreckiger Körper als Träger zur Bindung von Substanzen oder Molekülen für den Einsatz in enzymatischen, immunologischen, molekularbiologische oder genetischen Analyseverfahren, **dadurch gekennzeichnet, daß** er eine oder mehrere durchgängige Ausnehmungen besitzt, nicht ebenmäßig rund bzw. oval ist, sondern einem drei ober mehreckigen bzw. vieleckigen Körper entspricht. Wobei die Kantenlänge und -Zahl sowie Höhe des Körpers variabel ist.

2. Körper nach Anspruch 1, **dadurch gekennzeichnet, daß** eine oder mehrere durchgängige Ausnehmungen einem mehreckigen oder vieleckigen Querschnitt besitzt.

3. Körper nach Anspruch 1 bis 2, **dadurch gekennzeichnet, daß** dieser aus Celluose, Papier, Glas, Keramik, Metall oder künstlichen bzw. natürlichen Polymeren (z.B. Polyacrylamid, Nylon, Polyacryl, Zellulose, Polylysin, etc.) gefertigt sein kann.

4. Körper nach Anspruch 1 bis 3, **dadurch gekennzeichnet, daß** diese aus Papier, Glas, Keramik oder künstlichen bzw. natürlichen Polymeren (z.B. Polyacrylamid, Nylon, Polyacryl, Zellulose, Polylysin, etc.) gefertigt ist, in dem Metallpartikel eingelagert oder beschichtet sind oder diese elektromagnetische Eigenschaften haben.

5. Körper nach Anspruch 1 bis Anspruch 4, **dadurch gekennzeichnet, daß** der Hohlkörper aus lichtundurchlässigem oder lichtdurchlässigem Material gefertigt ist.

6. Verfahren mit den Körpern nach Anspruch 1 bis 4, **dadurch gekennzeichnet, daß** diese mit Hilfe einer Mechanik wie Greifer, Unterdrucksauger und/oder mit elektromagnetischen Verfahren transportiert werden und somit in automatisierten Analyseverfahren eingesetzt werden können.

7. Verfahren mit Körpern nach Anspruch 1 bis 5, **dadurch gekennzeichnet, daß** an den Körpern direkt keine Substanzen gebunden werden, diese aber mit einer Beschichtung auf deren Oberfläche versehen werden, welche aktiviert werden kann, so daß Moleküle aller Art gebunden werden können.

8. Körper nach Anspruch 1 bis 6, **dadurch gekennzeichnet, daß** diese als Reaktionskammer in Analysegeräte eingespannt werden.

9. Verfahren für Körper nach Anspruch 1 bis 5, aus Metallen oder künstlichen oder natürlichen Polymeren mit eingelagerten Metallpartikeln, **dadurch gekennzeichnet, daß** diese durch Elektromagnetismus bewegt werden können oder durch elektromagnetische Induktion erwärmt werden.
